(19) Europäisches Patentamt

European Patent Office

Office européen des brevets

(11) **EP 4 592 962 A1**

(12) **EUROPEAN PATENT APPLICATION**

(43) Date of publication:
**30.07.2025   Bulletin 2025/31**

(21) Application number: **24305152.1**

(22) Date of filing: **29.01.2024**

(51) International Patent Classification (IPC):
*G06T 15/08* (2011.01)       *G06T 19/20* (2011.01)

(52) Cooperative Patent Classification (CPC):
**G06T 15/08; G06T 19/20;** G06T 2210/41;
G06T 2219/2012

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Dassault Systèmes
78140 Vélizy-Villacoublay (FR)**

(72) Inventors:
• **ROULLET, Agathe
78140 VELIZY VILLACOUBLAY (FR)**
• **NDOKO, Arthur
78140 VELIZY VILLACOUBLAY (FR)**

(74) Representative: **Bandpay & Greuter
11 rue Christophe Colomb
75008 Paris (FR)**

(54)    **VOLUME RENDERING OF A SET OF MEDICAL IMAGES**

(57)    The disclosure notably relates to a computer-implemented method for volume rendering a set of medical images of a patient. The method comprises obtaining (S10) the set of medical images defining a voxel grid comprising voxels. The voxels are each associated to a value (hereinafter also referred to as "intensity value"). The method comprises segmenting (S20) the voxel grid defined by the set of medical images by associating to each voxel a respective label among a set of labels each corresponding to a respective region of interest of the patient. The method comprises modifying (S30) values of the voxel grid by adding to each value to be modified an offset value which depends on the respective label associated to the voxel. Such a method provides an improved solution for volume rendering a set of medical images of a patient.

```
┌─────────────────────────────────────────────┐
│ Obtaining the set of medical images defining │  ⌒ S10
│ a voxel grid comprising voxels, the voxels   │
│ being each associated to a value             │
└─────────────────────────────────────────────┘
                      │
                      ▼
┌─────────────────────────────────────────────┐
│ Segmenting the voxel grid defined by the set │  ⌒ S20
│ of medical images by associating to each     │
│ voxel a respective label among a set of      │
│ labels each corresponding to a respective    │
│ region of interest of the patient            │
└─────────────────────────────────────────────┘
                      │
                      ▼
┌─────────────────────────────────────────────┐
│ Modifying values of the voxel grid by adding │  ⌒ S30
│ to each value to be modified an offset value │
│ which depends on the respective label        │
│ associated to the voxel                      │
└─────────────────────────────────────────────┘
```

FIG. 1

## Description

### TECHNICAL FIELD

The disclosure relates to the field of computer programs and systems, and more specifically to a method, system and program for volume rendering a set of medical images of a patient.

### BACKGROUND

Several widely used medical imaging techniques such as Computed Tomography Scan (CT-scan) or MRI (Magnetic Resonance Imaging) provide 3D acquisitions of the anatomy. The output of these medical imaging exams are usually vertical stacks of 2D image slices that can be interpreted as volume representations of the patient's anatomy. Generating 3D visual rendering of these volumes has become key for a growing number of applications such as medical research, surgery planning, surgery guiding or patient communication.

In general, there are two main ways to represent 3D objects for visualization: volume representation and surface representation.

The most common one is surface representation that consists in representing the objects by their surfaces approximated by meshes of geometrical primitives such as triangles or quads. This representation is very sparse as it is hollow, only the borders of the object of interest are kept. Rasterization and ray-tracing algorithms enable to generate visualizations of these surfaces. For medical applications, a segmentation mask of the object to represent is necessary in order to define its borders, then distinctive colors or realistic textures are applied to the surface of each separate mesh to generate the final rendering. Alternatively, the surface can be defined as an iso-surface for a user-defined level of intensity. Surface representations are not well suited to represent anatomical structures that do not have well defined borders and to represent the intensity variations within the volume of the objects. For example, fine details such as thin blood vessels appear blurry in CT-Scans, they cannot be accurately rendered using surface representation.

The second, volume representation, is particularly adapted to 3D medical imaging. It consists in representing 3D objects by voxel grids filled with density and color values. The conversion of a 3D medical imaging volume to a volume representation is performed by mapping the intensity values of the capture to color and opacity values according to so-called transfer functions. Visualizations are then generated using a volume ray casting algorithm. Such visualizations are called direct volume rendering or simply volume rendering. Contrarily to surface representation, volume rendering enables to reveal smooth intensity variations across the tissues of the body.

One of the main limitations of known solutions for volume rendering is that they do not allow representing with distinctive appearances anatomical structures that share overlapping ranges of intensity values. Indeed, transfer functions are only dependent on intensity values, therefore they are spatially blind: different anatomical structures located at different locations with similar intensity values will have the same appearance (color and opacity). Moreover, they also do not allow visualizing anatomical structures when there are located behind tissues that have overlapping intensity values because of occlusion problems.

One solution is to combine volume rendering with surface representations for selected objects of interest. Indeed, surface representations are spatially aware, and each object surface can thus have its own texture or color. However, as mentioned above, surface representation is not suited to every anatomical structures. Moreover, it induces a loss of granularity compared to volume representations.

Within this context, there is still a need for an improved solution for volume rendering a set of medical images of a patient.

### SUMMARY

It is therefore provided a computer-implemented method for volume rendering a set of medical images of a patient. The method comprises obtaining the set of medical images defining a voxel grid comprising voxels. The voxels are each associated to a value. The method comprises segmenting the voxel grid defined by the set of medical images by associating to each voxel a respective label among a set of labels each corresponding to a respective region of interest of the patient. The method comprises modifying values of the voxel grid by adding to each value to be modified an offset value which depends on the respective label associated to the voxel.

The method may comprise one or more of the following:

- the offset value added to each value is equal to the result of a multiplication of the respective label associated to the voxel by a predetermined factor;
- the modifying comprises computing an offset voxel grid $V^m$ based on the following formula:

$$V^m = V + tS$$

wherein V is the voxel grid defined by the set of medical images, t is the predetermined factor and S is the segmented voxel grid;
- the method further comprises transmitting the computed offset voxel grid to a viewer;
- each segment is associated to a respective transfer function, the respective transfer function outputting, for each voxel of the segment to which the function is associated, a respective value for a set of at least two appearance parameters including a color parameter and an opacity parameter, the set of at least two appearance parameters optionally including at least one additional appearance parameter such as a metalness parameter and/or a rugosity parameter;
- one or more of the transfer functions are predetermined;
- one or more of the transfer functions are user-defined;
- the method further comprises:

  ◦ combining the respective transfer functions in a single piecewise transfer function; and
  ◦ transmitting the single piecewise transfer function to the viewer;

- the single piecewise transfer function is in the form:

$$\forall i \in \{0, ..., n_{objects}\}, \qquad f^m(v) = f_i(v - ti), \; if \; v \in [v_{min} + ti, v_{max} + ti].$$

wherein $f^m$ is the single piecewise transfer function, $f_i$ is the respective transfer function associated to the segment $i$, $v_{min}$ and $v_{max}$ are respectively the minimum and maximum values of the voxel grid and $t$ is the predetermined factor;
- the predetermined factor is higher than an amplitude of the values of the voxel grid;
- the regions of interest of the patient are selected among:

  ◦ one or more organs such as brain, heart, lungs, liver, pancreas, kidneys, skin and/or gut;
  ◦ one or more tissues such as epithelial tissues, connective tissues (e.g., adipose tissues), muscular tissues and/or nerve tissues;
  ◦ one or more vessels such as blood vessels; and/or
  ◦ one or more pathological forms such as tumors; and/or

- the set of medical images has been produced by a CT scanner or an MRI scanner.

It is further provided a computer program comprising instructions for performing the method.
It is further provided a computer readable storage medium having recorded thereon the computer program.
It is further provided a system comprising a processor coupled to a memory, the memory having recorded thereon the computer program.
The system may further comprise a viewer. The viewer may comprise a graphical user interface configured for displaying the volume rendering of the set of medical images. The viewer may be a medical imaging viewer able to perform direct volume rendering.
It is further provided a device comprising a data storage medium having recorded thereon the computer program. The device may form or serve as a non-transitory computer-readable medium, for example on a SaaS (Software as a service) or other server, or a cloud based platform, or the like. The device may alternatively comprise a processor coupled to the data storage medium. The device may thus form a computer system in whole or in part (*e.g.* the device is a subsystem of the overall system). The system may further comprise a viewer. The viewer may comprise a graphical user interface configured for displaying the single set of images.

**BRIEF DESCRIPTION OF THE DRAWINGS**

Non-limiting examples will now be described in reference to the accompanying drawings, where:

- FIG. 1 shows a flowchart of an example of the method;
- FIG. 2 shows an example of occlusion problem in a volume rendering using a known solution;
- FIG. 3 shows an example of the computed offset voxel grid;
- FIG. 4 illustrates another flowchart of an example of the method;
- FIGs. 5 and 6 illustrate examples of the single piecewise transfer function;
- FIGs. 7 and 8 show examples of volume rendering using the method; and

- FIG. 9 shows an example of the system.

**DETAILED DESCRIPTION**

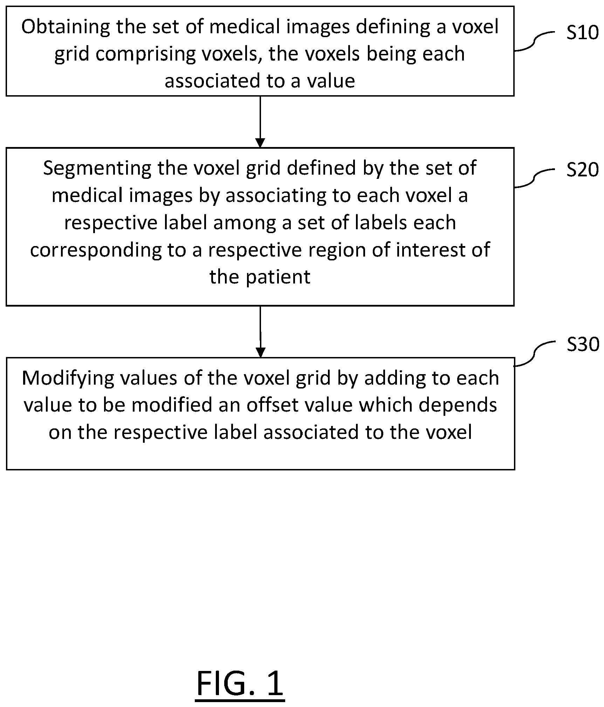

With reference to the flowchart of FIG. 1, it is proposed a computer-implemented method for volume rendering a set of medical images of a patient. The method comprises obtaining S10 the set of medical images defining a voxel grid comprising voxels. The voxels are each associated to a value (hereinafter also referred to as "intensity value"). The method comprises segmenting S20 the voxel grid defined by the set of medical images by associating to each voxel a respective label among a set of labels each corresponding to a respective region of interest of the patient. The method comprises modifying S30 values of the voxel grid by adding to each value to be modified an offset value which depends on the respective label associated to the voxel.

Such a method provides an improved solution for volume rendering a set of medical images of a patient.

Notably, the method allows volume rendering the set of medical images of the patient with arbitrary distinctive appearances for the different regions of interest. Such a volumetric representation reveals smooth intensity variations across body tissues, which is an improvement over surface representation solution. In particular, the volume rendering of the set of medical images allows revealing different tissues and anatomical structures.

Moreover, the method allows representing with distinctive appearances the different anatomical structures represented in the set of medical images. Indeed, the added offset values highlight the different regions of interests resulting from the segmentation S20, and therefore the different anatomical structures that these segments represent. In particular, the method allows representing with distinctive appearances anatomical structures, even when these anatomical structures share overlapping ranges of intensity values. The segmentation S20 allows distinguishing the different anatomical structures that the set of medical images represents by associating labels to the voxels. Based on these assigned labels, the method then allows distinguishing the different anatomical structures in the voxel values by adding offset values S30, thereby it allows representing with distinctive appearances these different anatomical structures that are represented in the set of medical images.

The method is for volume rendering the set of medical images of the patient. It means that the method may produce a new voxel grid (i.e., the said offset voxel grid) whose values have been modified with respect to the initial voxel grid defined by the obtained set of medical images. In particular, the values have been modified so as to highlight the different anatomical structures that are defined by the provided segmentation mask. It enables all the regions of interest revealed by the several sets of medical images to be visualized into a single set. The method may then comprise displaying this produced new voxel grid (e.g., using a 3D viewer as explained in the following). This display may be used by a medical practitioner (e.g., a doctor or nurse) so as to study the patient's area represented in the set of medical images, or to show it to the patient.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g., one for the program, and possibly one for the database).

The obtaining S10 of the set of medical images may comprise acquiring the set of medical images. For example, the set of medical images may be acquired by a CT-scan or any other imaging medical device (e.g., an MRI scan). In that case, the obtaining S10 may comprise the acquisition of the set of medical images using the CT-scan or the other imaging medical device. Alternatively, the set of medical images may have already been acquired at the time the method is executed. In that case, the obtaining S10 may comprise retrieving the already acquired set of medical images. For example, the set of medical images may have been recorded on a memory, and the obtaining S10 may comprise retrieving from the memory the acquired set of medical images.

The set of medical images may cover (i.e., images) an area of the body of the patient. The use of such a set of medical images for imaging an area of the body of the patient is well known. For example, it is known that a scanner-type examination may involve taking medical images regularly along (e.g., a part of) the patient's body, each representing a slice of the patient's body. Assembled together, these medical images may then be used to reconstruct, slice by slice, a 3D volume representing (e.g., the part of) the patient's body. The medical images of the set may thus together represent a 3D volume that includes the area of the patient. Each medical image may be a 2D image and may represent a respective slice along this 3D volume represented by the set. The 3D volume may thus be formed by assembling the successive slices represented by the different medical images of the set.

The set of medical images covers an area of the patient. In other words, the area of the patient is represented in at least of a portion (e.g., all) of the medical images of each set. The area covered by the set of medical images may be any area of the body of the patient. For example, the area of the body may include partially or totally the head, the neck, the trunk (thorax, abdomen and pelvis), one or both of the upper limbs and/or one or both of the lower limbs.

The set of medical images may define a voxel grid. This voxel grid may be aligned with a longitudinal direction of the area of the patient's body covered by the set of medical images. The voxel grid may comprise layers of voxels superimposed on one another along this longitudinal direction. Each voxel layer may comprise the voxels of the grid that are located at a same position along the longitudinal direction. Each voxel layer may represent a respective slice of the 3D volume represented by the set. Each medical image of the set may be perpendicular to this longitudinal direction. Each voxel may have a value which is defined by the set of medical images. In particular, each medical image may define the values of the voxels belonging to the voxel layer that is located at the same position along the longitudinal direction than the medical image. When the voxel layer and the medical image are superimposed, each voxel may have a value that corresponds to that in the portion of the medical image that the voxel includes.

The values within the voxel grid may be decimal numbers. The values within the voxel grid may be intensity values acquired during the acquisition of the set of medical images. When acquired by a CT-scan, the medical images of the set may be produced by sending X-rays through the human body. A resulting signal may be read and analyzed to reconstruct a dense volume of the body. In that case, the values of the voxel that are defined by the set of medical images may be Hounsfield Unit (HU) values. The HU is a relative quantitative measurement of radio density used by radiologists in the interpretation of computed tomography (CT) images. The values within the voxel grid may comprise at least two different values. For the voxels belonging to the background of the set of medical images (i.e., not included in the area of the patient), the values may be lower close to a predetermined value (e.g., - 1000, which represents the air).

The set of medical images may cover regions of interest. The regions of interest may be included in the area of the body of the patient covered by the set of medical images. Each region of interest may be a respective portion of this area of the patient's body. The regions of interest may each be any type of region of the human body that may be targeted by a type of medical imaging (i.e., whose appearance, content and/or shape can be revealed by this medical imaging). For example, the regions of interest may include one or more (e.g., portion of) organs such as brain, heart, lungs, liver, pancreas, kidneys, skin and/or gut. Alternatively or additionally, the regions of interest may include one or more tissues such as epithelial tissues, connective tissues (e.g., adipose tissues), muscular tissues and/or nerve tissues. Alternatively or additionally, the regions of interest may include one or more vessels such as blood vessels. Alternatively or additionally, the regions of interest may include one or more pathological forms such as tumors.

The set of medical images may cover at least one region of interest among all of the possible regions of interest listed above. It means that the intensity values within the voxel grid defined by the set of medical images may capture the content and/or shape of at least a portion of this at least one region of interest. For example, voxels belonging to a region of interest may have a value that varies within a certain range (depending, e.g., on the density at the voxel position in the region of interest). Voxels not belonging to a region of interest may have a predetermined value, which is that of the image background (e.g., corresponding to air).

The segmenting S20 of the set of medical images may comprise computing a segmentation mask of the voxel grid defined by the set of medical images. The segmentation mask is referred to as the "segmented voxel grid". The segmented voxel grid may have the same dimensions as the voxel grid defined by the set of medical images, but may comprise, for each voxel, a label indicating the region of interest to which the voxel belongs. The label is among the set of labels each corresponding to a respective region of interest of the patient. For example, the regions of interest may comprise $n_{objects}$ regions of interest (with $n_{objects}$ an integer), and the set of labels may be included $\{1, ... , n_{objects}\}$ (one for each region of interest). Additionally, the set of labels may include another label corresponding to the background (e.g., the label "0"). Each voxel $(z, y, x)$ of the segmented voxel grid may thus be filled with an integer value $S(z, y, x)$ ranging from 0 to $n_{objects}$, which is the number of segmented regions of interest that may be any type of anatomical structures listed above (e.g., tumor, fat or organs). $S(z, y, x) = i \in \{1, ... , n_{objects}\}$ may indicate that the voxel $(z, y, x)$ belongs to the region of interest labeled $i$. $S(z, y, x) = 0$ may indicate that the voxel $(z, y, x)$ belongs to the background.

The computing of the segmentation mask may be performed in any manner. For example, the computing of the segmentation mask may comprise creating an empty voxel grid having the same dimensions as the voxel grid defined by the set of medical images and filling each voxel of this empty voxel grid with label according to which region of interest the voxel belongs. The filling of the voxels with labels may comprise, for each region of interest covered in the set of medical images, detecting the voxels belonging to the region of interest, and filling the corresponding voxels in the empty voxel grid (i.e., located at the same position) with the label associated to the region of interest to which they belong.

The detecting of the voxels belonging to the region of interest may be performed based on a segmentation technique. For example, the detecting of the voxels belonging to the region of interest may be based a segmentation by thresholding based on intensity values within voxels. Alternatively, the detecting of the voxels belonging to the region of interest may be based on spatial variations in intensity values within voxels. A significant variation (e.g., above a given threshold between two voxels) may signify a change in the region of interest. The filing may then comprise filling the voxels detected as belonging to a same region of interest with the label associated to the region of interest to which they belong. For the remaining voxels (e.g., having values lower than a threshold), the filling may comprise filling the voxels of the empty voxel grid corresponding to these remaining voxels with the label associated to the background (e.g., the label "0").

The detecting of the voxels with labels may be performed automatically using a known algorithm, e.g., a neural network, for instance U-Net described in the paper by O. Ronneberger, P. Fischer, and T. Brox, "U-Net: Convolutional Networks for Biomedical Image Segmentation," in Medical Image Computing and Computer-Assisted Intervention - MICCAI 2015, N. Navab, J. Hornegger, W. M. Wells, and A. F. Frangi, Eds., in Lecture Notes in Computer Science. Cham: Springer International Publishing, 2015, pp. 234-241. doi: 10.1007/978-3-319-24574-4_28, which is incorporated herein by reference. U-Net has a classic automatic segmentation architecture. Alternatively, the detecting of the voxels with labels may be performed semi- automatically. For example, the filling of the voxels with labels may involve a user performing certain actions, for example to determine the outline of the regions of interest to be considered or to validate regions of interest that have been automatically detected by a known algorithm. For example, the determining of the outline of the regions of interest may be performed by a user. The determining may comprise displaying the set of medical images (e.g., each medical image successively) and selecting, through user interaction, the voxels belonging to the different regions of interest that are visible on the display (e.g., by defining the outline of each region of interest on each medical image).

The modifying S30 may comprise, for each voxel to be modified, adding to the value of the voxel the offset value which depends on the respective label associated to the voxel. Only a portion of the voxels may be modified. For example, the method may comprise modifying the values of the voxels that belong the regions of interest, and may comprise leaving unmodified the values of the voxels that belong to the background.

The offset value may depend on the region of interest in which the voxel is located. In the resulting voxel grid, each region of interests may include exclusively voxels having values spawning in respective (i.e., non-overlapping) ranges. For each voxel to be modified, the added offset value may be equal to the result of a multiplication of the respective label associated to the voxel by a predetermined factor. The predetermined factor may be the same for all the voxels. The predetermined factor may depend on the amplitude of the intensity values within the voxel grid defined by the set of medical images. For example, the predetermined factor may be strictly higher than the amplitude of the values within the voxel grid defined by the set of medical images. This predetermined factor may be manually defined. Alternatively, the factor may be automatically computed, for example, as the difference between the maximum and the minimum intensities (e.g., prior modifying step S30) in the voxel grid plus any positive value (e.g., 1).

The modifying S30 may be performed by considering all the voxels in succession one by one. For example, for each voxel, the modifying S30 may comprise identifying the label associated to the voxel and determining the offset value to be added based on the identified label (e.g., by multiplying the identified label with the previously predetermined factor). Then, the modifying S30 may comprise, for each voxel, attributing a new value to the voxel by adding the offset value determined for the voxel to the value of the voxel. When the voxel does not belong to a region of interest, the new value may be the same as the one the voxel already has. For example, for voxels belonging to the background, the label may be zero, which implies that the result of multiplying the identified label by the predetermined factor is also zero, and the value remains the same for each of these voxels.

Alternatively, the modifying S30 may be performed by considering all the voxels in a same mathematical operation. In that case, the modifying S30 may comprise computing an offset voxel grid $V^m$ based on the following formula:

$$V^m = V + tS$$

wherein V is the voxel grid defined by the set of medical images, t is the predetermined factor and S is the segmented voxel grid. The offset voxel grid may be the voxel grid with the modified values.

In examples, the method may comprise, after the modifying S30, rendering the voxel grid having the modified values (i.e., the offset voxel grid). The rendering of the offset voxel grid may comprise preparing data for 3D visualization of the offset voxel grid by a 3D viewer, e.g., a known 3D viewer such as 3D Slicer (described in the paper by A. Fedorov et al., "3D Slicer as an Image Computing Platform for the Quantitative Imaging Network," Magn Reson Imaging, vol. 30, no. 9, pp. 1323-1341, Nov. 2012, doi: 10.1016/j.mri.2012.05.001, which is incorporated herein by reference), ITK Snap (described in the paper by Paul A. Yushkevich, Joseph Piven, Heather Cody Hazlett, Rachel Gimpel Smith, Sean Ho, James C. Gee, and Guido Gerig. User-guided 3D active contour segmentation of anatomical structures: Significantly improved efficiency and reliability. Neuroimage 2006 Jul 1;31(3):1116-28, which is incorporated herein by reference) or OsiriX (described in the website page https://www.osirix-viewer.com/osirix/osirix-md/, which is incorporated herein by reference). The viewer may be a medical imaging viewer able to perform direct volume rendering. The prepared data may comprise the computed offset voxel grid. After the rendering, the method may comprise sending (or transmitting) the prepared data to the 3D viewer (i.e., including the computed offset voxel grid). The 3D viewer may be configured for displaying the offset voxel grid based on the data sent. In examples, the method may stop after the sending of the data to the 3D viewer. In other examples, the method may also comprise, after the sending of the data to the 3D viewer, the displaying of the resulting voxel grid by the 3D viewer based on the data sent. For example, the 3D viewer may comprise a graphical user interface, and the method may comprise the displaying of the resulting voxel grid on the graphical user interface.

The displaying of the offset voxel grid by the 3D viewer may be performed by applying a volume rendering algorithm (e.g. the ray casting algorithm). During the execution of the volume rendering algorithm, the intensity values of the offset voxel grid may be converted to at least two appearances values including (e.g., an RGB value) and an opacity value (e.g., a rate representing the opacity of the voxel in the resulting rendering) according to a transfer function (i.e., by applying the transfer function to the values of the offset voxel grid). These appearance values may further include additional values such as, for example, a metalness value and/or a rugosity value. The transfer function may be defined as the junction of multiple transfer functions forming a single piecewise transfer function. Each of these multiple transfer functions may be defined on the range of intensity values in the offset voxel grid associated with a region of interest or the background (i.e., the region outside any region of interest) resulting from the segmenting S20. The conversion of the intensity values of the offset grid according to the single piecewise transfer function enables to distinctively highlight the different regions of interest, that may strictly encompass anatomical structures, in the displaying of the voxel grid.

In examples, the applying of the volume rendering algorithm may be performed by the 3D viewer. In that case, the data sent to the 3D viewer may include the one or more transfer functions to be applied. The applying of the one or more transfer functions to the offset voxel grid may be performed by the 3D viewer. Each transfer function may take as input an intensity value of a given voxel and may output a respective value for each appearance parameter of the set for this given voxel. Each transfer function may be applied to a respective set of voxels. For example, each segment (or region of interest) may be associated to a respective transfer function, and this respective transfer function may be applied to the voxels of this segment. The segments are the segments obtained as a result of the segmenting S20 (i.e., the regions of interest). The assigning of the values may comprise, for each segment, applying the respective transfer function associated to the segment to the voxels belonging to the segment. It allows representing with distinctive appearances the different anatomical structures represented in the set of medical images. Indeed, each segment comprising voxels representing a same anatomical structure, the method allows applying a respective function to each segment of voxels representing a same anatomical structure, and therefore rendering each anatomical structure with a distinctive appearance.

In examples, one or more (e.g., all) of the multiple transfer functions may be user-defined. In that case, the method may comprise determining the one or more user-defined transfer functions by a user (e.g., a medical practitioner or an operator). The determining of each user-defined transfer function may comprise determining a graph of the transfer function. For example, the transfer function may be defined as a broken-line graph, and in that case, the determining the graph of the function may comprise adding, removing, and/or moving the breakpoints of the broken-line graph thanks to a digital user interface.

In examples, one or more (e.g., all) of the multiple transfer functions may be predetermined. In that case, the one or more predetermined transfer functions may have been determined prior to the executing of the method. For example, the shape of the function and/or its characteristic points may have been recorded on a memory, and the method may comprise retrieving, for each of the predetermined transfer functions, the shape of the function and/or its characteristic points from the memory.

In examples, prior to the sending of the data, the method may combine the respective transfer functions in a single piecewise transfer function. Each part of this single transfer function may correspond to a respective region of interest and may have its own color and intensity scale. For example, the single piecewise transfer function may be in the form:

$$\forall i \in \{0, \ldots, n_{objects}\}, \quad f^m(v) = f_i(v - ti), \ \ if \ v \in [v_{min} + ti, v_{max} + ti].$$

wherein $f^m$ is the single piecewise transfer function, $f_i$ is the respective transfer function associated to the segment $i$, $v_{min}$ and $v_{max}$ are respectively the minimum and maximum values of the voxel grid and t is the predetermined factor. The single transfer function renders the method compatible with known generic direct volume rendering tools.

Each respective transfer function $f_i$ may take as input an intensity value and may outputs a respective value for each appearance parameters of the set base on the inputted intensity value. For example, each respective transfer function $f_i$ may be in the form:

$$\forall v \in [v_{min}, v_{max}], f_i(v) = (\boldsymbol{c}, \sigma, \boldsymbol{p})$$

wherein $\boldsymbol{c}$ is a RGB color, $\sigma$ is an opacity value and $\boldsymbol{p}$ includes one or more values of potential one or more additional appearance parameters (e.g., including a metalness parameter, a specularity parameter and/or a roughness parameter, e.g., as described in https://en.wikipedia.org/wiki/Specular highlight). $v_{max}$ and $v_{min}$ may be respectively the maximum and the minimum intensity values within the voxel grid.

Then, the method may comprise transmitting the single piecewise transfer function to the viewer. For example, the single piecewise transfer function may be included in the data sent to the 3D viewer. The sending of the data to the 3D viewer may be performed in any manner. For example, the method may be executed on a computer on which the 3D viewer may be installed. The sending of the data may be performed using this connection. Then, the viewer may be configured to display the computed offset voxel grid based on the transmitted single piecewise transfer function (e.g., using a known volume ray casting algorithm).

Volume rendering designates the process of generating a 2D image corresponding to a view of a 3D scene when the underlying geometry of the scene is densely represented by 3D matrix (i.e., a voxel grid) whose values describe the local appearance properties of the volume. Common volume rendering algorithms include the ray casting or the maximum intensity projection algorithms. Volume rendering differs from surface rendering, which rather designates the process of generating a 2D image corresponding to a view of a 3D scene when the underlying geometry of the scene is sparsely represented by the surfaces of the objects it contains. These surfaces are usually decomposed into tileable primitives (triangles, quads) forming so-called meshes. Common surface rendering algorithms include the rasterization or the ray tracing algorithms. Contrary to surface rendering, volume rendering enables to account for opacity variations along the depth of the scene's elements. By allowing the performing a volume rendering, the method therefore improves the rendering of the area of the patient.

In examples, the method may comprise using the voxel grid with the modified values (i.e., the offset voxel grid) for preparing a surgery for the patient. For example, the method may be included in a surgery preparation process, which may comprise, after performing the method, preparing the surgery for the patient based on the offset voxel grid. The preparing of the surgery may comprise sending the offset voxel grid to a 3D viewer for displaying the offset voxel grid (e.g., as discussed above) and determining the surgical operations to be performed based on the displayed offset voxel grid (e.g., identifying the areas to be treated, determining the operations to be performed on these areas and/or determining the tool paths to be used to perform these operations). Alternatively or additionally, the method may comprise using the offset voxel grid for communicating with the patient. For example, the method may comprise sending the offset voxel grid to a 3D viewer for displaying the offset voxel grid to the patient (e.g., as discussed above) so that a medical practitioner (e.g., a doctor or nurse) may explain a condition visible in the displayed offset voxel grid to the patient. By enabling the displaying of the offset voxel grid to the patient, the method also improves surgical preparation and/or patient communication. Indeed, the method allows representing with distinctive appearances the different anatomical structures represented in the set of medical images, which is particularly useful for preparing a surgical procedure and/or communicating with a patient.

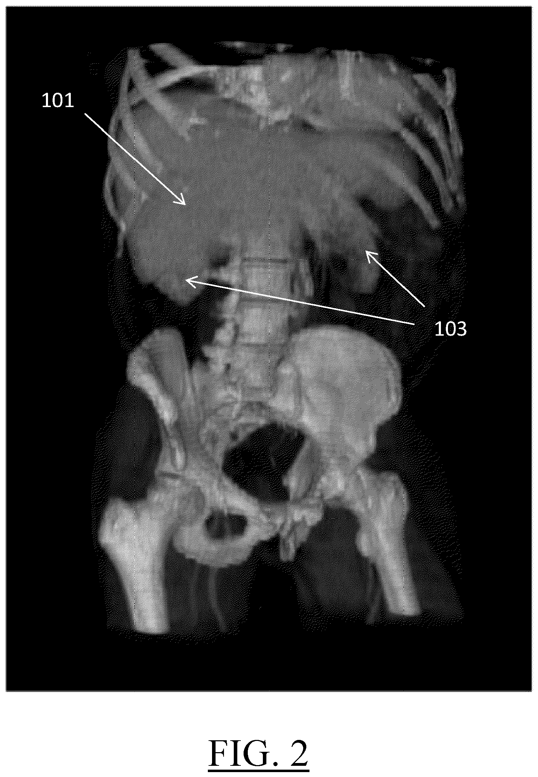

With reference to the FIGs. 2 to 9, an example of implementation of the method is now described.

In known solutions for volume rendering, intensity values of the medical imaging (e.g., Hounsefield Unit for CT-Scans) are converted to color and opacity values according to transfer function (also called colormap or look-up table). Depending on the chosen transfer function, the direct volume rendering reveals different tissues and anatomical structures. However, only a single transfer function can be used for the entire volume at once. Indeed, transfer functions are only dependent on intensity values, therefore they are spatially blind. In other words, transfer functions do not depend on the position of the voxel. Different anatomical structures located at different locations with similar intensities will have the same appearance (color and opacity).

Consequently, different anatomical structures cannot be visualized in the volume rendering because of occlusion problems (e.g., visceral fat is usually hidden by the skin in the volume rendering of a CT scan). More generally, it is not possible to render anatomical structures such as abdomen organs or different muscles with specific distinctive colors since they share overlapping ranges of intensities. For example, in FIG. 2, it is hard to distinguish the liver 101 from the kidney 103 because they have similar intensity values.

The method solves these occlusion problems by making the volume rendering spatially aware. In particular, the method requires very little coding and can be easily embedded in existing modern volume rendering pipelines.

Explanations of the terms used are now provided.

A CT-scan (Computed Tomography Scan) is a specific type of medical images produced by sending X-rays through the human body. The signal is read and analyzed to reconstruct a dense volume of the body.

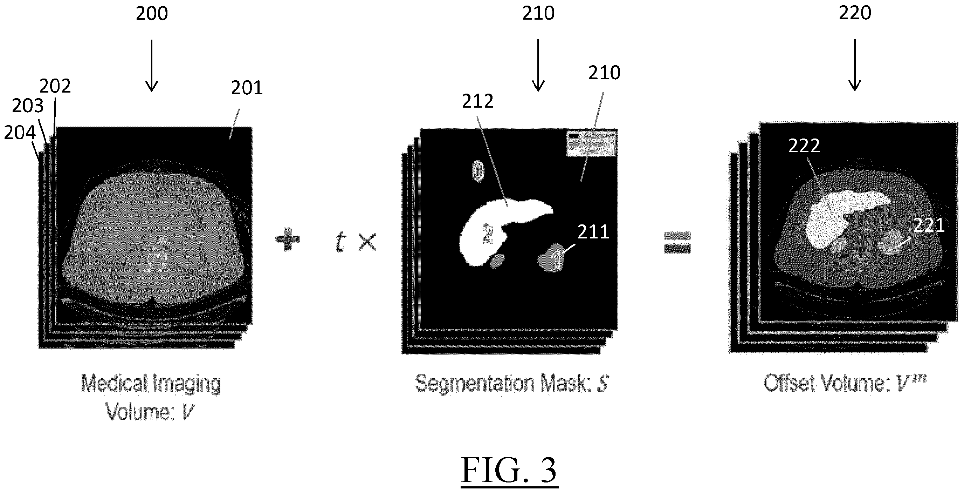

The Hounsfield unit (HU) is a relative quantitative measurement of radio density used by radiologists in the interpretation of computed tomography (CT) images (see, e.g., https://www.ncbi.nlm.nih.gov/books/NBK547721/). FIG. 3 shows an example of Hounsfield scale of CT scan. It gives example of range corresponding to each tissue (Bones: 400-1000 HU, soft tissues: 40-80 HU, ...).

A set of medical images may be a set of CT-scan images acquired at the same time in a single acquisition. They may define a voxel grid of HU values representing a patient's anatomy.

A region of interest (ROI) is an anatomical structure that the method aims at highlighting. For example, the region of interest may be an anatomical structure comprising a contrast product.

A transfer function (also called look-up table (LUT) or colormaps) is a function configured for mapping intensity values of medical imaging volume to color and opacity values in order to enable the volume rendering.

Direct volume rendering (or volume rendering) designates the process of generating 2D images from 3D volume data. Direct volume rendering methods are the ones requiring the conversion of the source volume into a 3D grid of color and opacity values thanks to a transfer function.

Ray casting is the baseline direct volume rendering algorithm. It consists in casting ray of sight from the observation position toward the volume. Color and opacity values are sampled along these rays. The color of each pixel of the resulting 2D image is obtained as a mixture of the colors of the points sampled on its associated rays of sight weighted by the opacity values.

A spatially blind function is a function that is not dependent on spatial information such as the location.

A spatially aware function is a function that is dependent on spatial information contrary to spatially blind functions.

FIG. 3 shows an example of the computed offset voxel grid.

The figure shows the set of medical images 200 taken as input by the method (obtained at step S10). The set of medical images defines a volume grid $V$ of dimensions $(D, H, W)$ holding the intensity data of a 3D medical imaging acquisition with $D$ the number of acquisition slices (i.e., the number of medical images of the set), and $H$ and $W$ respectively the height and width of each slice. The values within $V$ (referred to as $V$ values) range between $v_{min}$ and $v_{max}$. The amplitude of V values is denoted $v_{amp}$ and is equal to $v_{amp} = v_{max} - v_{min}$.

The figure shows the segmented voxel grid 210, which is a volume grid S of dimensions $(D, H, W)$ representing a segmentation mask of V created manually or automatically. S is filled with integer values ranging from 0 to $n_{objects}$, the number of segmented objects of interest that can be any type of anatomical structures (e.g. tumor, fat, organs etc.). $S(z, y, x) = i \in \{1, \dots, n_{objects}\}$ indicates that the voxel $(z, y, x)$ of V belongs to the object labeled $i$. $S(z, y, x) = 0$ indicates that the voxel $(z, y, x)$ of V belongs to the background.

In this example, the set of medical images comprises two regions of interest. In the segmented voxel grid 210, the voxels 211 belonging to the first region of interest are associated to the label "1" (the kidneys in this example). The voxels 212 belonging to the second region of interest are associated to the label "2" (the liver in this example). The voxels 210 belonging to the background are associated to the label "0".

The figure also shows the resulting offset voxel grid 220. The method computes the offset voxel grid 220 by adding the result of the multiplication of the segmented voxel grid 210 by the predetermined factor t to the initial voxel grid 200. The figure shows that the resulting offset voxel grid 220 have intensity values that are ranged in non-overlapping ranges for the first and second regions of interest (221 and 222). It allows highlighting these two different regions of interest in the final rendering.

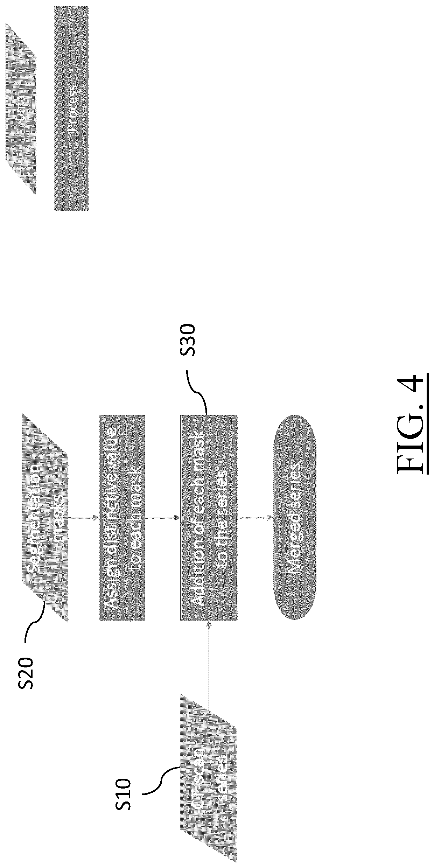

FIG. 4 illustrates another flowchart of an example of the method. In this example, the method comprises the following steps.

In a first step, the method comprises determining an offset $t > v_{amp}$ (e.g., $t = 5000$ for standard CT-Scans).

In a second step (at run time), the method comprises computing an offset volume $V^m = V + tS$ (as explained in reference to FIG. 3). The computing of the offset volume comprises the obtaining S10 of the set of medical images, the segmenting S20 of the voxel grid and the modifying S30 of the values of the voxel grid, thereby obtaining the offset volume $V^m$.

In a third step, the method comprises designing or choosing by a user the presets transfer functions $f_i$ specific to each object $i$ (including the background) such as:

$$\forall v \in [v_{min}, v_{max}], f_i(v) = (\boldsymbol{c}, \sigma, \boldsymbol{p})$$

wherein $\boldsymbol{c}$ is a RGB color, $\sigma$ is an opacity value and $\boldsymbol{p}$ parameters of potential other appearance properties (e.g., specularity or roughness). This step may be performed interactively, for example, using a medical imaging viewer (such as slicer, which is described in https://www.slicer.org).

In a fourth step, the method comprises combining the segmented object transfer function into a single piecewise transfer function $f^m$ defined by part and adapted to the modified $V^m$ such as:

$$\forall i \in \{0, \dots, n_{objects}\}, \qquad f^m(v) = f_i(v - ti), \ \ if \ v \in [v_{min} + ti, v_{max} + ti].$$

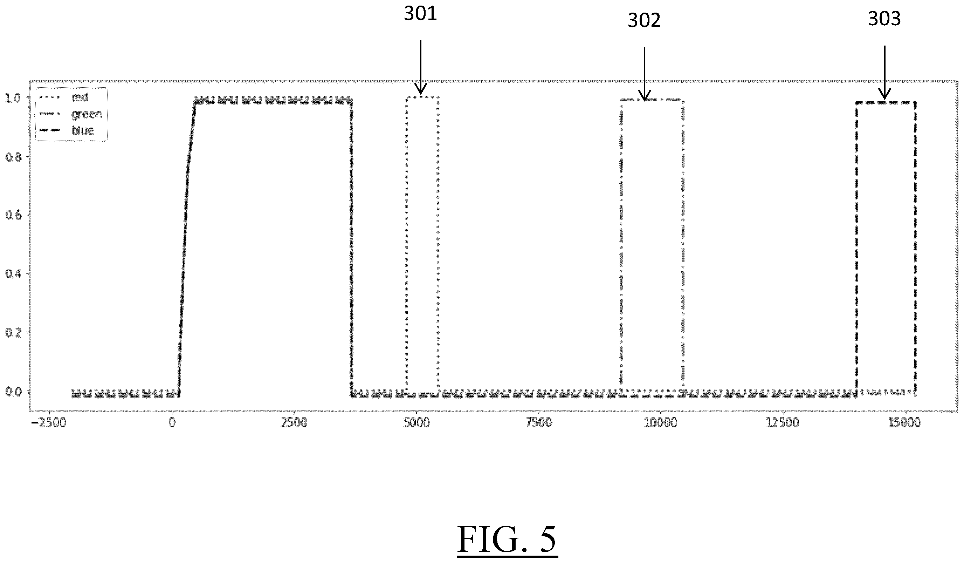

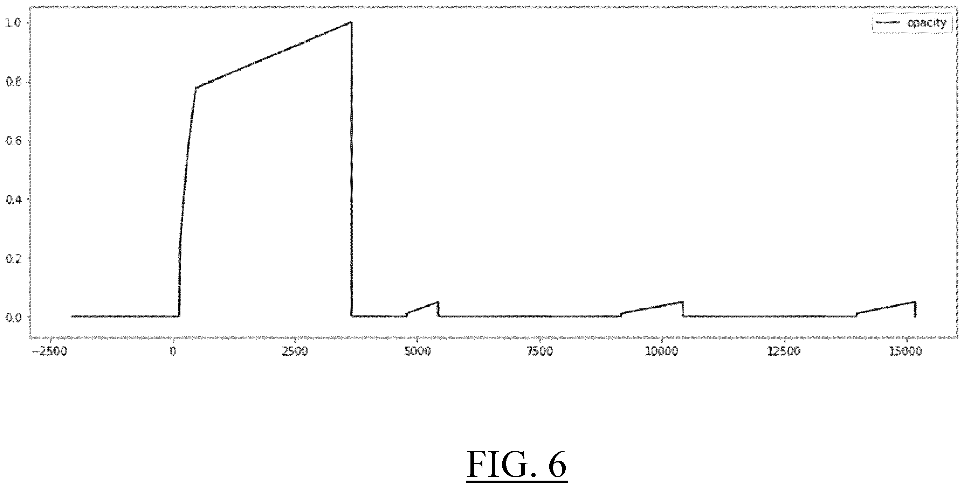

Thus, if the voxel $(z, y, x)$ belongs to the object i, then $f^m(V^m(z, y, x)) = f_i(V(z, y, x))$. FIGs. 5 and 6 illustrate an example of a piecewise transfer function.

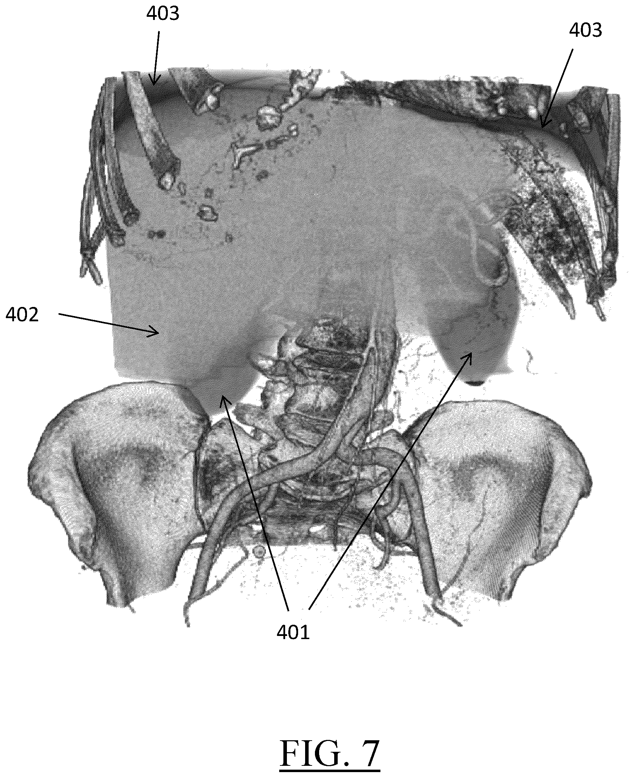

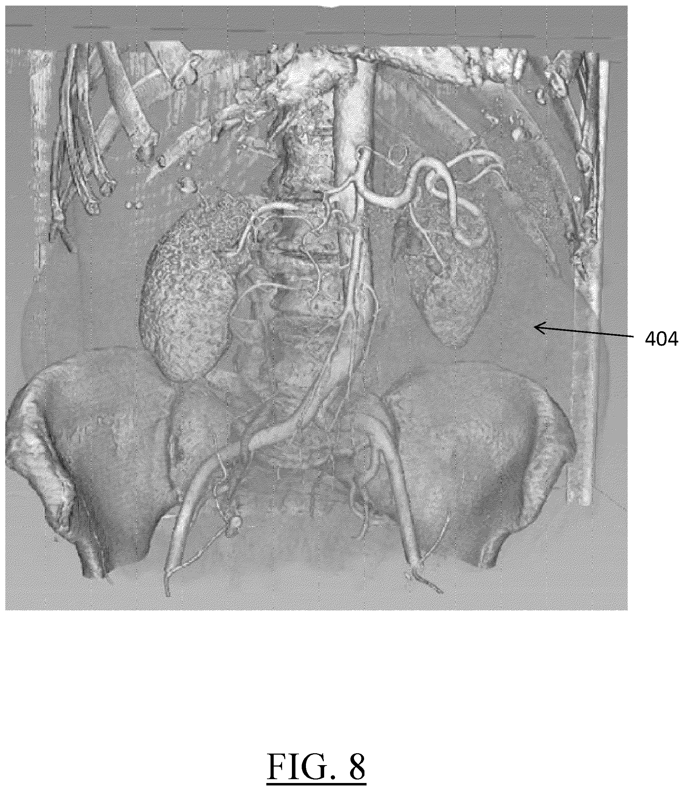

In a fifth step, the method comprises transmitting the modified volume $V^m$ and the transfer function $f^m$ to generic volume rendering tools (such as the Open3D library described in http://www.open3d.org/docs/release/# or the VTK library described in https://vtk.org/) to perform the volume ray casting algorithm in order to obtain the desired resulting visualization (see FIGs. 7 and 8).

FIGs. 5 and 6 illustrate examples of the single piecewise transfer function $f^m$. The FIG. 5 shows, in a graph, the RGB values outputted by the resulting single piecewise transfer function $f^m$ as a function of the modified intensity values. In this example, the method computes the single piecewise transfer function $f^m$ outputting the three RGBcolor channels: red, green and blue. The first peak 301 of the graph associated with the color "red" corresponds to the region of interest "kidneys". The second peak 302 of the graph associated with the color "green" corresponds to the region of interest "liver". The third peak 303 of the graph associated with the color "blue" corresponds to the region of interest "lungs". The FIG. 6 shows, in a graph, the opacity values outputted by the resulting single piecewise transfer function $f^m$ as a function of the modified intensity values. The first peak corresponds to the non-segmented part of the scan, and each of the next peaks corresponds to a segmented region (kidneys, liver and lungs as discussed in reference to FIG. 5). The resulting single piecewise transfer function $f^m$ can be decomposed into several (e.g. four in the example) transfer functions that each contributes within a respective range of intensity values (e.g. separated by an offset of 5000 HU in the example) and corresponds to a region of the body delimited by the segmentation mask (the non-segmented region, the kidneys, the liver and the lungs in the example), which allows highlighting distinctively the different regions of interest in the final rendering.

FIGs. 7 and 8 show examples of volume rendering using the method. In particular, FIG. 7 shows the resulting volume rendering obtained by the method for an example of CT-Scan and using the transfer function illustrated in FIGs. 5 and 6. The method enables to assign distinctive colors to liver 402, kidneys 401 and lungs 403 according to a segmentation mask (green, red and blue). In particular, the method allows clearly rendering the lungs 403, which are particularly difficult to render in known volume rendering solutions because their density values are close to air. FIG. 8 shows the resulting volume rendering obtained by the method for an example of a CT-Scan enriched with a segmentation mask of the visceral fat 404.

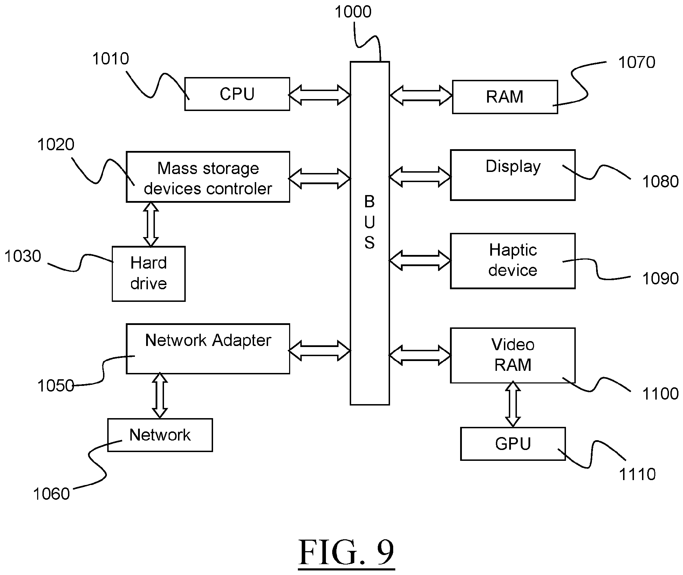

FIG. 9 shows an example of the system, wherein the system is a client computer system, e.g. a workstation of a user.

The client computer of the example comprises a central processing unit (CPU) 1010 connected to an internal communication BUS 1000, a random-access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

**Claims**

1. A computer-implemented method for volume rendering a set of medical images of a patient, the method comprising:

- obtaining (S210) the set of medical images defining a voxel grid comprising voxels, the voxels being each associated to a value;
- segmenting (S20) the voxel grid defined by the set of medical images by associating to each voxel a respective label among a set of labels each corresponding to a respective region of interest of the patient; and
- modifying (S30) values of the voxel grid by adding to each value to be modified an offset value which depends on the respective label associated to the voxel.

2. The method of claim 1, wherein the offset value added to each value is equal to the result of a multiplication of the respective label associated to the voxel by a predetermined factor.

3. The method of claim 1 or 2, wherein the modifying comprises computing an offset voxel grid $V^m$ based on the following formula:

$$V^m = V + tS$$

wherein $V$ is the voxel grid defined by the set of medical images, $t$ is the predetermined factor and $S$ is the segmented voxel grid;
and wherein the method further comprises transmitting the computed offset voxel grid to a viewer.

4. The method of any one of claims 1 to 3, wherein each segment is associated to a respective transfer function, the respective transfer function outputting, for each voxel of the segment to which the function is associated, a respective value for a set of at least two appearance parameters including a color parameter and an opacity parameter, the set of at least two appearance parameters optionally including at least one additional appearance parameter such as a metalness parameter and/or a rugosity parameter.

5. The method of claim 4, wherein one or more of the transfer functions are predetermined.

6. The method of claim 4 or 5, wherein one or more of the transfer functions are user-defined.

7. The method of any one of claims 4 to 6, the method further comprising:

- combining the respective transfer functions in a single piecewise transfer function; and
- transmitting the single piecewise transfer function to the viewer.

8. The method of claim 7, wherein the single piecewise transfer function is in the form:

$$\forall i \in \{0, \ldots, n_{objects}\}, \quad f^m(v) = f_i(v - ti), \; if \; v \in [v_{min} + ti, v_{max} + ti].$$

wherein $f^m$ is the single piecewise transfer function, $f_i$ is the respective transfer function associated to the segment $i$, $v_{min}$ and $v_{max}$ are respectively the minimum and maximum values of the voxel grid and t is the predetermined factor.

9. The method of any one of claims 2 to 8, wherein the predetermined factor is higher than an amplitude of the values of the voxel grid.

10. The method of any one of claims 1 to 9, wherein the regions of interest of the patient are selected among:

- one or more organs such as brain, heart, lungs, liver, pancreas, kidneys, skin and/or gut;
- one or more tissues such as epithelial tissues, connective tissues (e.g., adipose tissues), muscular tissues and/or nerve tissues;
- one or more vessels such as blood vessels; and/or
- one or more pathological forms such as tumors.

11. The method of any one of claims 1 to 10, wherein the set of medical images has been produced by a CT scanner or an MRI scanner.

12. A computer program comprising instructions for performing the method of any of claims 1 to 11.

13. A computer readable storage medium having recorded thereon the computer program of claim 12.

14. A system comprising a processor coupled to a memory, the memory having recorded thereon the computer program of claim 12.

15. The system of claim 14, further comprising a viewer, the viewer comprising a graphical user interface configured for displaying the volume rendering of the set of medical images.

Obtaining the set of medical images defining a voxel grid comprising voxels, the voxels being each associated to a value — S10

Segmenting the voxel grid defined by the set of medical images by associating to each voxel a respective label among a set of labels each corresponding to a respective region of interest of the patient — S20

Modifying values of the voxel grid by adding to each value to be modified an offset value which depends on the respective label associated to the voxel — S30

# FIG. 1

FIG. 2

Medical Imaging
Volume: $V$

$+$   $t\times$

Segmentation Mask: $S$

$=$

Offset Volume: $V^m$

<u>FIG. 3</u>

FIG. 4

FIG. 5

FIG. 6

EP 4 592 962 A1

FIG. 7

404

## FIG. 8

**FIG. 9**

Europäisches
Patentamt

European
Patent Office

Office européen
des brevets

# EUROPEAN SEARCH REPORT

**Application Number**

EP 24 30 5152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Bruckner Stefan: "Efficient Volume Visualization of Large Medical Datasets", Master's Thesis, 2004, pages i-iv, 1-111, XP093178004, Institute of Computer Graphics and Algorithms, Vienna University of Technology, Austria Retrieved from the Internet: URL:https://vis.uib.no/wp-content/papercite-data/pdfs/Bruckner-2004-EVV-Thesis.pdf * section 3.2.2 on pages 28-29 * | 1-15 | INV. G06T15/08 G06T19/20 |
| A,D | RONNEBERGER OLAF ET AL: "U-Net: Convolutional Networks for Biomedical Image Segmentation", 18 November 2015 (2015-11-18), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 234 - 241, XP047337272, ISBN: 978-3-540-74549-5 [retrieved on 2015-11-18] * abstract * | 1-15 | |
| A,D | ANDRIY FEDOROV ET AL: "3D Slicer as an image computing platform for the Quantitative Imaging Network", MAGNETIC RESONANCE IMAGING, vol. 30, no. 9, November 2012 (2012-11), pages 1323-1341, XP055572591, TARRYTOWN, NY, US ISSN: 0730-725X, DOI: 10.1016/j.mri.2012.05.001 * abstract * | 1-15 | |

TECHNICAL FIELDS
SEARCHED (IPC)

G06T

-/--

The present search report has been drawn up for all claims

| Place of search | Date of completion of the search | Examiner |
|---|---|---|
| Berlin | 24 June 2024 | Bouchaâla, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | YUSHKEVICH PAUL A. ET AL: "User-guided 3D active contour segmentation of anatomical structures: Significantly improved efficiency and reliability", NEUROIMAGE, vol. 31, no. 3, July 2006 (2006-07), pages 1116-1128, XP093177993, AMSTERDAM, NL ISSN: 1053-8119, DOI: 10.1016/j.neuroimage.2006.01.015 * abstract * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Berlin | 24 June 2024 | Bouchaâla, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................
& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- U-Net: Convolutional Networks for Biomedical Image Segmentation. **O. RONNEBERGER** ; **P. FISCHER** ; **T. BROX**. Medical Image Computing and Computer-Assisted Intervention - MICCAI 2015. Springer International Publishing, 2015, 234-241
- **A. FEDOROV et al.** 3D Slicer as an Image Computing Platform for the Quantitative Imaging Network. *Magn Reson Imaging*, November 2012, vol. 30 (9), 1323-1341

- **PAUL A. YUSHKEVICH** ; **JOSEPH PIVEN** ; **HEATHER CODY HAZLETT** ; **RACHEL GIMPEL SMITH** ; **SEAN HO** ; **JAMES C. GEE** ; **GUIDO GERIG.** User-guided 3D active contour segmentation of anatomical structures: Significantly improved efficiency and reliability.. *Neuroimage*, 01 July 2006, vol. 31 (3), 1116-28